# EUROPEAN PATENT SPECIFICATION

(11) **EP 2 452 654 B1**
(45) Date of publication and mention of the grant of the patent: **01.05.2013**
(21) Application number: 10710064.6
(22) Date of filing: 18.02.2010
(51) Int. Cl.: A61F 5/02, A61F 5/03

(54) **COMBINED CLASP FOR MULTI-BAND ORTHOPAEDIC ELEMENTS**
KOMBIKLAMMER FÜR ORTHOPÄDISCHE MULTIBANDELEMENTE
BOUCLE COMBINÉE POUR ÉLÉMENTS ORTHOPÉDIQUES MULTIBANDES

(43) Date of publication of application: 16.05.2012
(73) Proprietor: Prim, S.A., 28938 Mostoles (ES)
(72) Inventor: MEIJIDE GARCIA, José luis, E-28938 Mostoles (ES)
(74) Representative: Monzón de la Flor, Luis Miguel
(86) International application number: PCT/ES2010/070087
(87) International publication number: WO 2011/101505

(56) References cited:
- WO-A1-2010/114807
- US-A- 2 053 600
- US-A1- 2003 050 585
- US-A1- 2005 251 074

## Description

### OBJECT OF THE INVENTION

The object of this invention is a combined buckle for multi-band orthopedic elements, where the orthopedic element has been specially thought of and designed for fastening and fixing to any part of the body, be this trunk, upper limbs, lower limbs, etc.

It is characterized by the special configuration and design of the combined buckle that is the object of the invention, which is made in such a way that the manpower costs necessary to fix each of the loops is reduced, since all of them are fixed simultaneously. On the other hand, it improves and increases the fixing of the loops and finally, as a result of the design of each of the individual buckles that form part of the combined buckle, twists and kinks along the edges of the tightening straps are prevented.

Therefore, this invention is confined to orthopedic products designed and thought of for fixing and protecting a certain part of the body and particularly amongst the construction accessories, specifically each of the loops fixed on the orthopedic element for the elastic tightening straps to pass through.

### BACKGROUND OF THE INVENTION

In general, multi-band orthopedic elements have a series of tightening straps that can be made from elastic or another material and that allow a certain amount of pressure to be used and fixed, in order to achieve the pressure and immobilization of a certain area of the body. These tapes are fixed at one of their ends to the actual structure of the orthopedic element, whilst the other end is fixed to an adhesive end, fixed over a wide area of the orthopedic element. The tapes which are aimed at exerting a strong pressure in the area of the orthopedic element where they do not pass are made to go through some loops that are fixed by a tape to the actual structure of the orthopedic element.

The loops through which the tightening straps must pass are individual and are fixed to the structure of the orthopedic element by way of a fastening tape that is retained when it goes through the loop. This fastening tape is sewn to the actual structure of the orthopedic element.

Each of the loops is individually fixed and given that on each edge of the orthopedic element there is an average of five loops, the operation must be repeated ten times. Therefore, this fastening process starts with an available loop, then a fastening tape is passed through the loop and the ends of the tape are laid out in such a way that the loop is perfectly positioned both horizontally and vertically and is subsequently fixed by sewing the loop's fastening tape.

Once one loop has been attached, the operation must be repeated with as many loops as are being used, which means an important investment in manpower, as well as the fact that the perfect layout and alignment of the loops cannot be guaranteed.

In the state of the art it is known the patent US 20050251074 A in which it is disclosed a string arrangement for a detachment type waist protecting belt to hold the vertebra region of a vertebra related patient. The string arrangement enables a separate fastening of the upper portion and lower portion of the belt to form a saddle like shape that fits the contour of the waist of an individual patient dynamically with or without the extra support of a frame

Therefore, the aim of this invention is to improve the layout process of the loops, by developing a combined buckle such as the one that is the object of the invention, which means a clear reduction in time and an improvement in the construction processes.

### DESCRIPTION OF THE INVENTION

The object of the invention is a combined buckle for multi-band orthopedic elements, such as for example corsets, girdles, padded socks, knee pads and all those orthopedic elements that need a series of loops for some tightening straps to pass through.

Specifically, the combined buckle that is the object of the invention basically consists of a series of buckles laid out on a shared joining piece of the buckles forming a single piece, made in a resistant material and that can be perforated by a needle for sewing.

On one of the sides of the shared joining piece of the buckles, the buckles are laid out, aligned and joined to the aforementioned shared piece by a neck joint, which is sufficiently wide and thick to guarantee the join of each buckle to the shared piece.

The purpose for which the invention was designed is achieved thanks to the configuration of the piece that is the object of the invention, formed by a shared joining piece on which there are a series of buckles.

On the one hand, the necessary manpower costs for fixing the loops are reduced, since all the buckles that form part of the combined buckle are fixed simultaneously, without it being necessary to interrupt the sewing tasks to lay out and subsequently sew each one of the fastening tapes of the individual buckles.

On the other hand, it improves and increases the fixing of the buckles, since the fastening seam takes place on the actual shared joining piece to the orthopedic element and each of the buckles has a neck joint with the shared joining piece that is sufficiently wide and thick to bear the pressures to which it could be subjected.

Finally, as a result of the design of the space inbetween of each of the buckles, twists and kinks along the edges of the tightening straps are prevented.

As a complementary step to the abovementioned construction method, the shared joining piece of the buckles can at least have a thickening along its entire length, in such a way that at the same time as being used as a reinforcement for the piece itself, it may also be used as a stay for the orthopedic element or for the corset, as the piece remains hidden and sewn into the actual structure. In general, as was normal procedure prior to this application, a series of reinforcement stays are usually laid out in the area where the buckles are fastened. Now this combined buckle piece, reinforced at the shared joining piece achieves a double purpose: that of giving greater structural resistance to the combined buckle piece and that of fitting reinforcement stays to the actual orthopedic element.

### EXPLANATION OF THE FIGURES

To complement the description that is about to be made and in order to aid greater understanding of its characteristics, this descriptive report is accompanied by a set of plans showing the most significant details of the invention in the figures in an illustrative and non-limiting way.
Figure 1 shows a detailed representation of an orthopedic corset on which it is possible to lay out a combined buckle such as the one that is the object of the invention.
Figure 2 shows a detailed representation of the combined buckle that is the object of the invention, where its construction details may be appreciated.
Figure 3 shows a detail of how the combined buckle is joined to the corset and the layout of the tightening straps.

### PREFERRED PRODUCTION OF THE INVENTION

In view of the figures, a preferred production method for the proposed invention is now described.

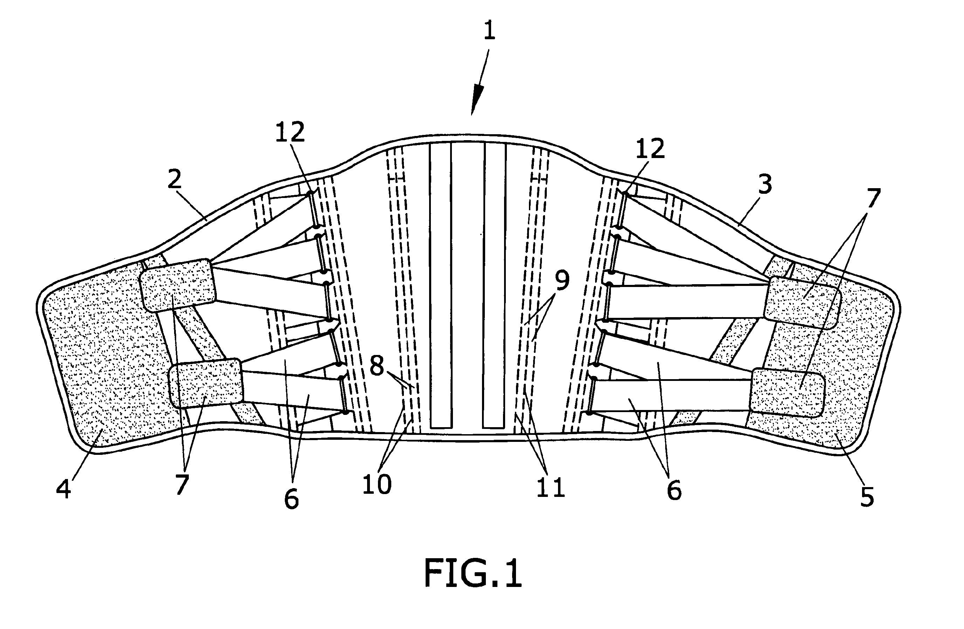

In figure 1 a representation of a corset is shown, where the actual central body (1) of the corset should be mentioned, on the ends of each side extensions emerge (2) and (3) for closure, where some fastening areas (4) and (5) of the ends of the tightening straps (6) are defined. The tightening straps (6) on the fastening areas (4) and (5) are fixed using some complementary elastic loops and hooks.

The tightening straps (6) are passed through the buckles included in the combined buckle (12) that is the object of the invention, being fixed at one of their ends to the corset itself, (this fastening has not been shown), whilst the other end has been fastened onto a fixing piece (7) that is used to give pressure and fix the tapes on the fastening areas (4) and (5).

In addition to this, the corset has a series of structural reinforcements using stays (8) and (9) housed in the spaces defined by some lines of overstitching (10) and (11) respectively and made on the actual corset itself, in order to immobilize the stays (8) and (9), the spaces where the stays (8) and (9) are housed are reinforced with pocket-shaped tapes.

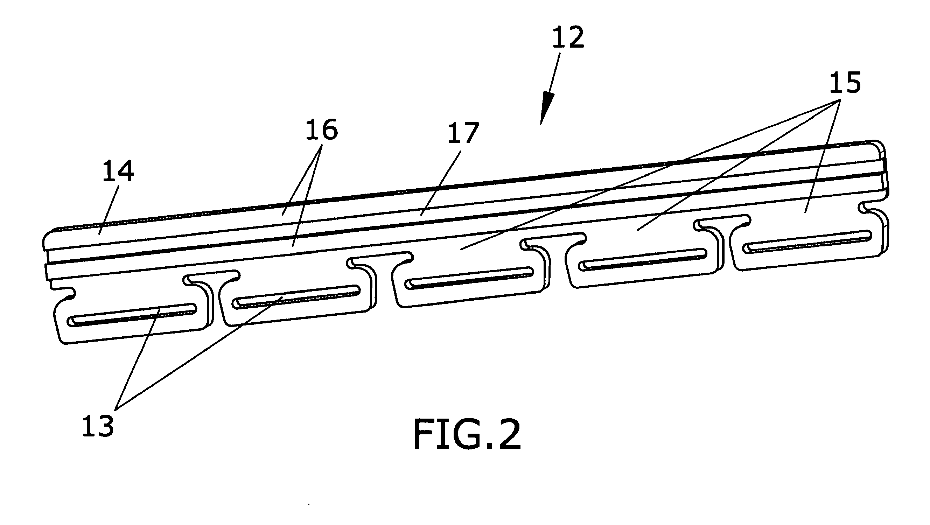

In figure 2 it may be seen how the combined buckle (12) has a shared joining piece (14) where at least on one of its sides there is a series of individual buckles (13) laid out, joined to the shared piece (14) by way of a neck joint (15).

The neck joint is sufficiently wide and thick to guarantee the structural resistance of the entire piece when faced with the different pressures to which it is going to be subjected.

In addition to this, the shared joining piece (14) has at least two or more reinforcements (16), which once the combined buckle has been drawn and fixed by sewing, may be used as reinforcement to the actual combined buckle itself and also as a stay.

According to the invention, the shared joining piece (14) has two or more lengthwise reinforcements (16) separated with a space between them (17).

The layout of the individual buckles (13) can be on one or on both sides of the shared joining piece (14), that is to say, either a single combined buckle or a double combined buckle may be used.

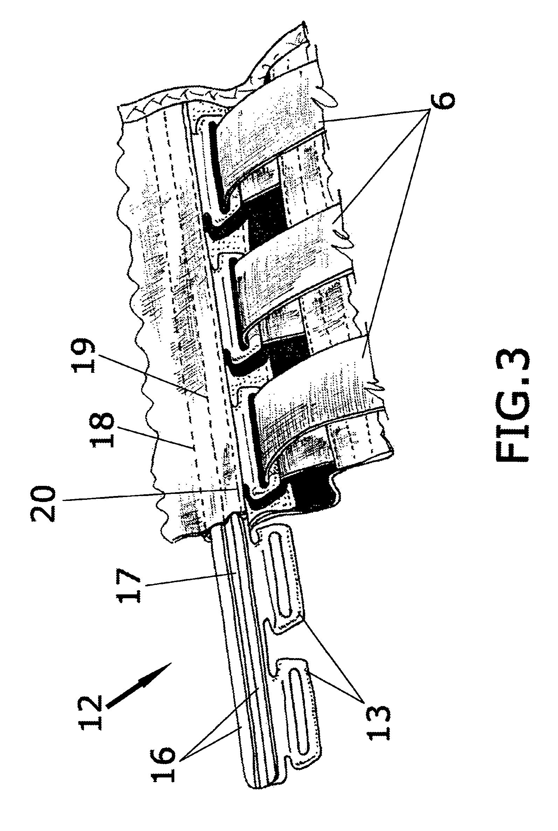

Therefore, in figure 3, it may be seen how the fixing of the entire piece on the corset is carried out, which means the fixing of each of the individual buckles through which the tightening straps must pass.

In the aforementioned figure 3, it may partially be seen how the combined buckle (12) that has two reinforcements (16), separated by an intermediate space (17) is fixed. With a first rear line of stitching (18) made along the free lengthwise edge of the combined buckle (12), a second line of stitching (19) that is made on the intermediate space (17) that separates both reinforcements and a third line of sewing or front line of sewing (20) that passes through the neck joint (15) of each individual buckle (13) with the shared joining piece (14), presenting the specific point that the three lines of stitching are carried out simultaneously, which drastically reduces the time necessary to fix the loops used until now, as well as the improvement in the alignment of the individual buckles of the combined buckle.

Thanks to the described piece, in a quick, simple and efficient way a series of buckles can be made available through which the tightening straps will pass, as well as reinforcing the corset itself, in the case that the shared joining piece were to have a series of reinforcements.

The use of the combined buckle that is the object of the invention can be applicable for all those multi-band orthopedic products that require fastening by tightening straps, such as orthopedic corsets, knee pads, ankle support pads, padded socks, girdles, items called "Walkers", which are elements that cover the foot and lower part of the leg to the knee designed to give stability and warmth to the ankle.

The essential nature of this invention is not altered by variations in materials, shape, size and layout of the component elements, described in a non-limiting way, this being sufficient for its reproduction by an expert.

## Claims

1. A combined buckle for orthopedic elements of the ones through which some tightening straps (6) are passed that are included on the orthopedic element, wherein the combined buckle comprises a shared joining piece (14) where on at least one of its sides there is a series of individual buckles (13) laid out, each joined to the shared piece (14) by a neck joint (15), **characterized in that** the shared joining piece (14) has two or more lengthwise reinforcements (16) separated by an intermediate space (17).

2. Combination of an orthopedic element and a combined buckle as defined in the antecedent claim characterized because the fixing of the combined buckle (14) to the orthopedic element has a first rear line of sewing (18) along the lengthwise free edge of the combined buckle (12), a second line of sewing (19) that is made in the intermediate space (17) that separates both reinforcements and a third line of sewing or front line of sewing (20) that goes through the neck joint (15) of each individual buckle (13) with the shared joining piece (14), allowing all the seams to be sewn simultaneously.

3. Use of a combined buckle according to claim 1 **characterized in that** it is used for orthopedic elements such as orthopedic corsets, knee pads, ankle support pads, padded socks, girdles or "Walkers".

## Patentansprüche

1. Eine Kombiklammer für orthopädische Elemente, von der Art, durch die einige Straffbänder (6) geführt werden, die zu dem orthopädischen Element gehören, wobei die Kombiklammer ein geteiltes Verbindungsstück (14) umfasst, wobei auf mindestens einer ihrer Seiten eine Reihe einzelner Klammern (13) angeordnet ist, wobei jede mit dem geteilten Stück (14) über ein Halsgelenk (15) verbunden ist, **dadurch gekennzeichnet, dass** das geteilte Verbindungsstück (14) über zwei oder mehrere Längsverstärkungen (16) verfügt, die durch einen Zwischenraum (17) voneinander getrennt sind.

2. Kombination eines orthopädischen Elements mit einer Kombiklammer gemäß der Definition im vorigen Anspruch, **dadurch gekennzeichnet, dass** die Befestigung der Kombiklammer (14) an dem orthopädischen Element eine erste rückwärtige Nähnaht (18) entlang der freien Ecke der Kombiklammer (12), eine zweite Nähnaht (19), die im Zwischenraum (17) verläuft, welcher die beiden Verstärkungen voneinander trennt, und eine dritte Nähnaht bzw. vordere Nähnaht (20), die über das Halsgelenk (15) jeder einzelnen Klammer (13) verläuft, hat, mit dem geteilten Verbindungsstück (14), wobei das gleichzeitige Nähen aller Nähte ermöglicht wird.

3. Verwendung einer Kombiklammer nach Anspruch 1, **dadurch gekennzeichnet, dass** sie für orthopädische Elemente, wie zum Beispiel orthopädische Korsetts, Kniepolster, Knöchelstützpolster, gepolsterte Socken, Gürtel und Gehhilfen verwendet wird.

## Revendications

1. Une boucle combinée pour éléments orthopédiques du type de celles à travers lesquelles on fait passer des sangles de serrage (6) qui sont incluses dans l'élément orthopédique, où la boucle combinée comprend une pièce d'assemblage partagée (14) où sur au moins l'un de ses côtés se trouve une série de boucles séparées (13) disposées, chacune étant assemblée à la pièce partagée (14) par un joint à col (15), **caractérisée en ce que** la pièce d'assemblage partagée (14) possède deux ou plusieurs renforcements longitudinaux (16) séparés par un espace intermédiaire (17).

2. Combinaison d'un élément orthopédique et d'une boucle combinée telle que définie dans la revendication précédente caractérisée parce que la fixation de la boucle combinée (14) à l'élément orthopédique possède une première ligne arrière d'assemblage par couture (18) le long du bord libre longitudinal de la boucle combinée (12), une deuxième ligne d'assemblage par couture (19) qui est exécutée dans l'espace intermédiaire (17) qui sépare les deux renforcements et une troisième ligne d'assemblage par couture ou ligne avant d'assemblage par couture (20) qui traverse le joint à col (15) de chaque boucle séparée (13) avec la pièce d'assemblage partagée (14), permettant à toutes les coutures d'être cousues simultanément.

3. Utilisation d'une boucle combinée selon la revendication 1 **caractérisée en ce qu'**elle est utilisée pour des éléments orthopédiques tels que des corsets orthopédiques, genouillères, protège-chevilles, chaussettes rembourrées, gaines ou chaussures de marche.
